Europäisches Patentamt

(19) European Patent Office

Office européen des brevets

(11) Publication number: **0 255 809**
A1

# (12) EUROPEAN PATENT APPLICATION

(21) Application number: **87830283.5**

(22) Date of filing: **24.07.87**

(51) Int. Cl.⁴: **A 61 B 5/10**

(30) Priority: **01.08.86 IT 3599886**

(43) Date of publication of application:
**10.02.88 Bulletin 88/06**

(84) Designated Contracting States:
**BE CH DE ES FR GB LI NL**

(71) Applicant: **Vanin, Mario**
**Via Magenta 14**
**I-00185 Roma RM (IT)**

**Ayad, Amale**
**84 Rue du Château**
**F-92100 Boulogne (FR)**

(72) Inventor: **Vanin, Mario**
**Via Magenta 14**
**I-00185 Roma RM (IT)**

**Ayad, Amale**
**84 Rue du Château**
**F-92100 Boulogne (FR)**

(74) Representative: **Tonon, Gilberto et al**
**c/o Società Italiana Brevetti Piazza Poli 42**
**I-00187 Roma (IT)**

(54) A device for the the measurement of the elasticity of the human skin.

(57) A device for the measurement of the elasticity of the human skin, by which a negative pressure is created in an area of the skin up to reaching a predetermined value and the temporary deformation of the skin itself is measured; by returning the negative pressure to the zero value, the deformation of the skin in correspondence with that value is measured, i.e. the permanent deformation of the skin.

EP 0 255 809 A1

## Description

### "A DEVICE FOR THE MEASUREMENT OF THE ELASTICITY OF THE HUMAN SKIN"

In the field of dermatology and of cosmetics it is often necessary to perform a measurement of the elasticity of the skin, both for prescribing specific treatments and for the selection of appropriate creams.

Apparatus are known which, for the determination of the above mentioned parameter, exert a predetermined pressure established by a small weight laid over an area of the skin and that according to such pressure measure the temporary deformation of the skin and, moreover, after the elimination of such pressure taking away the weight, measure the permanent deformation of the skin. Apparatus are also known which, for measuring the elasticity of the skin, exert a torsional stress on the intended skin area.

The above mentioned apparatus according to the prior art, show, however, several drawbacks deriving from an excessive sophistication and mechanical complexity to which one should add a high cost, a low precision and a complexity of use by insufficiently skilled operators.

The aim of the present invention is to provide a device for the measurement of the elasticity of the skin having an extremely simple structure, easy to operate, having a low cost, but, however, capable of providing the required data with a surprising precision.

According to the present invention, in place of exert ing a pressure or torsion on the skin, as happens in all the similar apparatus according to the prior art, a negative pressure is created in an area of the skin up to reaching a predetermined value and the temporary deformation of the skin itself is measured. By returning the negative pressure to the zero value, the deformation of the skin in correspondence with that value is measured, i.e. the permanent deformation.

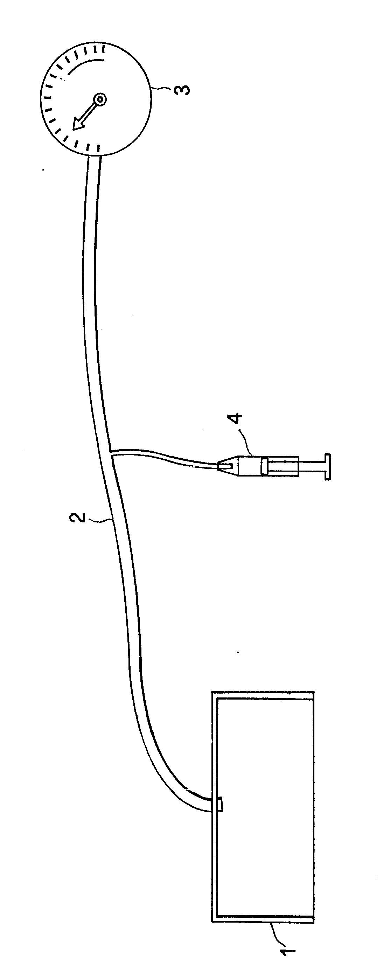

The present invention will be better illustrated by the disclosure of a preferred embodiment thereof, given by way of a non limiting example, and by referring to the single figure of the attached drawing which constitutes a schematical view of the device in question.

With reference to the above mentioned figure, in 1 a suction cup is shown, shaped as an inverted cup, sealingly connected at its top to a first end of a pipe 2, the other end of which is sealingly connected to a precision pressure gauge.

Between the suction cup 1 and the pressure gauge 3, a syringe 4 is inserted into the pipe 2, said syringe 4 being provided with a suitable graduated scale, for creating in the extraction stroke of its plunger a predetermined negative pressure within the suction cup 1.

In its operation, the suction cup 1 is placed into contact with the selected area of the skin to be subjected to an elasticity measurement, obtaining the required sealing by means of an adhesive cream coated on said application area. Then a suction is performed by means of the syringe 4 creating thus a negative pressure in the interior of the cup 1, the value of which suitably predetermined, for instance 10 mm Hg., is read on the dial of the pressure gauge 3. In correspondence with said predetermined value of the negative pressure, on the graduated scale of the syringe 3 the amount of the extraction stroke of the plunger is read.

Said reading will be proportional to the temporary deformation, shaped as a bulge, which the skin undergoes as a consequence of the negative pressure within the cup 1. Then the plunger of the syringe 4 is pushed towards the starting position until on the pressure gauge 3 a zero value of negative pressure is read, to which on the graduated scale of the syringe 4 a given residual stroke of the plunger will correspond, the value of which is proportional to the permanent deformation of the skin.

The present invention is not limited to the described embodiment, but extends to any modification thereof.

## Claims

1. A device for the measurement of the elasticity of the skin comprising: a suction cup to be sealingly applied on a selected area of the skin where the elasticity measurement is to be made; a pressure measurement instrument connected to the suction cup; suction means, connected with said suction cup, for creating in said suction cup a negative pressure up to a predetermined value readable by means of said pressure measurement instrument and provided with measuring means in such a way that, at said predetermined value of the negative pressure, it is possible to determine the volume of air sucked by said suction means or a corresponding parameter, which is proportional to the temporary deformation of the skin in said area where the suction cup is applied and is subjected to said negative pressure, and in such a way that, by bringing said suction means back towards the start position up to a zero value of said negative pressure, it is possible to detect the volume of the possible residue of sucked air, which is proportional to the permanent deformation of the skin.

2. A device according to claim 1, wherein said suction means are plunger-type suction means provided with a graduated scale so that, at said predetermined value of the negative pressure, the amount of the displacement stroke of the plunger of said suction means, proportional to said temporary deformation of the skin, is determined and, by pushing said plunger towards the start position up to a zero value of said negative pressure, it is possible to detect the amount of the possible residue of said

**0 255 809**

displacement stroke of said plunger, which is proportional to the permanent deformation of the skin.

3. A device according to claim 1 or 2, wherein said pressure measurement instrument is a precision pressure gauge.

4. A device according to claim 2, wherein said plunger-type suction means comprise a syringe.

5. A device according to any of the claims 1 to 4, wherein said predetermined value of the negative pressure is preferably comprised between 5 and 10 mm Hg.

6. A device according to any of the preceding claims, wherein the sealing application of said suction cup is performed by coating beforehand said preselected area of the skin with an adhesive cream.

0255809

1

2

3

4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A- 733 686 (M. CHOPIN)<br>* page 2, lines 72-103; page 3, lines 62-78; figure 1 * | 1,4 | A 61 B 5/10 |
| A | | 2,3 | |
| | --- | | |
| A | GB-A-2 068 567 (L'OREAL)<br>* page 1, lines 28-52; page 2, lines 22-51; figure 1 * | 1,2 | |
| | ----- | | |

TECHNICAL FIELDS SEARCHED (Int. Cl.4)

A 61 B 5/00

The present search report has been drawn up for all claims

| Place of search | Date of completion of the search | Examiner |
|---|---|---|
| BERLIN | 05-11-1987 | WEIHS J.A. |